# EUROPEAN PATENT APPLICATION

(11) **EP 2 397 467 A1**
(43) Date of publication of application: **21.12.2011**
(21) Application number: 10165530.6
(22) Date of filing: 10.06.2010
(51) Int. Cl.: C07D 215/18, C07D 215/20, A01N 43/42

(54) **Quinoline derivatives as fungicides**

(71) Applicant: Syngenta Participations AG, 4058 Basel (CH)
(72) Inventor: The designation of the inventor has not yet been filed
(74) Representative: Herrmann, Jörg

(57) **Abstract**

Compounds of the general formula 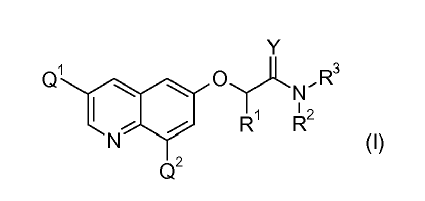 wherein the substituents are as defined in claim 1, are useful as fungicides.

## Description

This invention relates to novel acid amides, processes for preparing them, to compositions containing them and to methods of using them to combat fungi, especially fungal infections of plants.

Certain acid amide derivatives and their use as fungicides are disclosed, for example, in WO09/030467 and WO09/030469.

The present invention is concerned with the provision of particular substituted acid amides for use mainly as plant fungicides.

Thus, according to the present invention there is provided a compound of the general formula (I) 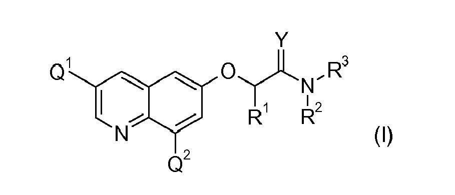 wherein
Q¹ is methyl, ethyl, n-propyl, isopropyl, vinyl or propenyl;
Q² is hydrogen, fluoro, chloro or methyl;
R¹ is ethyl, methoxy or methylthio;
R² is hydrogen or methyl;
R³ is -CR⁴R⁵R⁶_{;}
R⁴ and R⁵, independently of each other, are hydrogen, methyl, ethyl, isopropyl, t-butyl, vinyl, ethynyl, cyano or methoxymethyl, or
R⁴ and R⁵ together with the carbon atom to which they are attached form a 3- to 5-membered carbocyclic ring, which is optionally substituted by methyl;
R⁶ is hydrogen, formyl, methyl, ethyl, 2-fluoroethyl, vinyl, ethynyl, prop-1-ynyl, but-1-ynyl, cyano, cyclopropyl, methoxymethyl, ethoxymethyl, -CH=NOMe, -CH=NOEt or - C=CCH₂OMe; and
Y is oxygen or sulfur;
or a salt or a N-oxide thereof.

The compounds of the invention contain at least one asymmetric carbon atom and therefore may exist as enantiomers, as pairs of diastereoisomers or as mixtures of such.

Furthermore, isomerism around the C=N double bond of compounds of the invention can exist thereby leading to stereochemically isomeric forms of compounds of the general formula (I). In cases where the compounds of the invention exist as the E and Z isomers, the invention includes individual isomers as well as smixtures thereof.

Compounds of general formula (I) can therefore exist as racemates, diastereoisomers, or single enantiomers, and the invention includes all possible isomers or isomer mixtures in all proportions. It is to be expected that for any given compound, one isomer may be more fungicidally active than another. N-oxides of the compounds of the formula (I) preferably denote the N-oxides formed, for example, when Ar is heteroaryl such as a quinolinyl or quinazolinyl moiety.

The salts which the compounds of the formula I can form are preferably those formed by interaction of these compounds with acids. The term "acid" comprises mineral acids such as hydrogen halides, sulphuric acid, phosphoric acid etc. as well as organic acids, preferably the commonly used alkanoic acids, for example formic acid, acetic acid and propionic acid.

N-oxides are preferably compounds of the formula I with an oxygen atom at the nitrogen atom of the quinoline ring.

The carbocyclic rings preferably contain 3 or 4 carbon atoms and are cyclopropyl or cyclobutyl. Optional substituents on these rings comprise halo, alkyl, alkoxyalkyl, alkenyl, alkynyl, haloalkyl, cyano, hydroxyalkyl, alkoxy, optionally substituted aryl and optionally substituted heteroaryl. Halo includes fluoro, chloro, bromo and iodo.

Of particular interest are those compounds of the formula I, wherein Q¹ is methyl, ethyl, vinyl or propenyl, in particular methyl or vinyl.

Preferably, Q² is hydrogen or methyl.
R¹ is preferably methylthio.
R² is preferably hydrogen.
R⁴ and R⁵, independently of each other, are preferably methyl, ethyl, vinyl, ethynyl or cyano, or, also preferred, one of R⁴ and R⁵ is methyl and the other of R⁴ and R⁵ is ethyl, vinyl, ethynyl or cyano.

Preferably, R⁴ and R⁵ together with the carbon atom to which they are attached form cyclobutyl.
R⁶ is preferably methyl, ethynyl, -CH=NOMe or -C=CCH₂OMe; and
Y is preferably oxygen.

In a particularly preferred group of compounds of the formula I, Q¹ and Q² are methyl and R¹ is methylthio.

In another preferred group of compounds of formula I, wherein Q¹ is methyl or vinyl; Q² is hydrogen, chloro or methyl; R¹ is ethyl, methoxy or methylthio; R² is hydrogen; R³ is - CR R R ; R⁴ and R⁵, independently of each other, are hydrogen, methyl, ethyl, isopropyl, t-butyl, vinyl, ethynyl, cyano or methoxymethyl, or R⁴ and R⁵ together with the carbon atom to which they are attached form a cyclobutyl ring, which is optionally substituted by methyl; R⁶ is hydrogen, methyl, ethyl, vinyl, ethynyl, prop-1-ynyl, cyano, methoxymethyl, -CH=NOMe or -C=CCH₂OMe; and Y is oxygen.

Compounds that form part of the invention are illustrated in Tables 1 to 48 below.

Compounds of the formula 1:

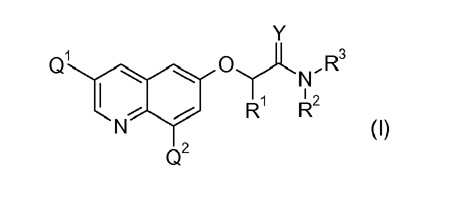

**Table 1 The compounds of Table 1 are of the general formula (I) where Q¹ is methyl, Q² is hydrogen, Y is oxygen, R¹ is ethyl, R² is hydrogen and R³ (i.e. ―CR⁴R⁵R⁶ ) has the values given in the table.**

| Compound No. | R⁴ | R⁵ | R⁶ |
|---|---|---|---|
| 1 | H | CH₃ | CH₃ |
| 2 | CH₃ | CH₃ | CH₃ |
| 3 | H | CH₃ | C=CH |
| 4 | CH₃ | CH₃ | C=CH |
| 5 | H | CH₃ | CH=NOCH₃ |
| 6 | CH₃ | CH₃ | CH=NOCH₃ |
| 7 | H | CH₃ | C=CCH₃ |
| 8 | CH₃ | CH₃ | C=CCH₃ |
| 9 | H | CH₃ | C=CCH₂OCH₃ |
| 10 | CH₃ | CH₃ | C=CCH₂OCH₃ |
| 11 | H | CH₃ | CH(CH₃)₂ |
| 12 | CH₃ | CH₃ | CH(CH₃)₂ |
| 13 | H | CH₃ | CH₂OCH₃ |
| 14 | CH₃ | CH₃ | CH₂OCH₃ |
| 15 | H | CH₃ | ▷ |
| 16 | CH₃ | CH₃ | ▷ |
| 17 | H | CH₂CH₃ | ▷ |
| 18 | CH₃ | CH₂CH₃ | ▷ |
| 19 | H | CN | ▷ |
| 20 | CH₃ | CN | ▷ |
| 21 | H | C=CH | ▷ |
| 22 | CH₃ | C=CH | ▷ |
| 23 | H | CH₃ | CH₂OCH₂CH₃ |
| 24 | CH₃ | CH₃ | CH₂OCH₂CH₃ |
| 25 | H | CN | CH₂OCH₃ |
| 26 | CH₃ | CN | CH₂OCH₃ |
| 27 | H | C=CH | CH₂OCH₃ |
| 28 | CH₃ | C=CH | CH₂OCH₃ |
| 29 | H | CH₃ | 2-pyridyl |
| 30 | CH₃ | CH₃ | 2-pyridyl |
| 31 | H | H | ▷ |
| 32 | H | CH₃ | CH₂CH₂F |
| 33 | CH₃ | CH₃ | CH₂CH₂F |
| 34 | H | C=CH | C≡CH |
| 35 | CH₃ | C≡CH | C≡CH |
| 36 | H | CN | CH=NOCH₃ |
| 37 | CH₃ | CN | CH=NOCH₃ |
| 38 | H | C=CH | CH=NOCH₃ |
| 39 | CH₃ | C≡CH | CH=NOCH₃ |
| 40 | H | CH₃ | CN |
| 41 | CH₃ | CH₃ | CN |
| 42 | H | CN | CN |
| 43 | CH₃ | CN | CN |
| 44 | H | C=CH | CN |
| 45 | CH₃ | C≡CH | CN |
| 46 | C≡CH | C≡CH | CH₂OCH₃ |
| 47 | C≡CH | CH₂OCH₃ | CH=NOCH₃ |
| 48 | H | CH=CH₂ | C≡CH |
| 49 | CH₃ | CH=CH₂ | C≡CH |
| 50 | CH₃ | CH₂OCH₃ | CH=NOCH₃ |
| 51 | H | CH=CH₂ | CH₂OCH₃ |
| 52 | CH₃ | CH=CH₂ | CH₂OCH₃ |
| 53 | CH₂CH₂CH₂ | | H |
| 54 | CH₂CH₂CH₂ | | CH₃ |
| 55 | CH₂CH₂CH₂ | | CH=NOH |
| 56 | CH₂CH₂CH₂ | | CH=NOCH₃ |
| 57 | CH₂CH₂CH₂ | | C≡CH |
| 58 | CH₂CH₂CH₂ | | CN |
| 59 | CH₂CH₂CH₂ | | CHO |
| 60 | CH₂OCH₂ | | CH₃ |

| | | | |
|---|---|---|---|
| ▷ = cyclopropyl | | | |

### Table 2

The compounds of Table 2 are of the general formula (I) where Q¹ is methyl, Q² is fluoro, Y is oxygen, R¹ is ethyl, R² is hydrogen and R³ has the values given in Table 1.

### Table 3

The compounds of Table 3 are of the general formula (I) where Q¹ is methyl, Q² is chloro, Y is oxygen, R¹ is ethyl, R² is hydrogen and R³ has the values given in Table 1.

### Table 4

The compounds of Table 4 are of the general formula (I) where Q¹ is methyl, Q² is methyl, Y is oxygen, R¹ is ethyl, R² is hydrogen and R³ has the values given in Table 1.

### Table 5

The compounds of Table 5 are of the general formula (I) where Q¹ is methyl, Q² is hydrogen, Y is oxygen, R¹ is methoxy, R² is hydrogen and R³ has the values given in Table 1.

### Table 6

The compounds of Table 6 are of the general formula (I) where Q¹ is methyl, Q² is fluoro, Y is oxygen, R¹ is methoxy, R² is hydrogen and R³ has the values given in Table 1.

### Table 7

The compounds of Table 7 are of the general formula (I) where Q¹ is methyl, Q² is chloro, Y is oxygen, R¹ is methoxy, R² is hydrogen and R³ has the values given in Table 1.

### Table 8

The compounds of Table 8 are of the general formula (I) where Q¹ is methyl, Q² is methyl, Y is oxygen, R¹ is methoxy, R² is hydrogen and R³ has the values given in Table 1.

### Table 9

The compounds of Table 9 are of the general formula (I) where Q¹ is methyl, Q² is hydrogen, Y is oxygen, R¹ is methylthio, R² is hydrogen and R³ has the values given in Table 1.

### Table 10

The compounds of Table 10 are of the general formula (I) where Q¹ is methyl, Q² is fluoro, Y is oxygen, R¹ is methylthio, R² is hydrogen and R³ has the values given in Table 1.

### Table 11

The compounds of Table 11 are of the general formula (I) where Q¹ is methyl, Q² is chloro, Y is oxygen, R¹ is methylthio, R² is hydrogen and R³ has the values given in Table 1.

### Table 12

The compounds of Table 12 are of the general formula (I) where Q¹ is methyl, Q² is methyl, Y is oxygen, R¹ is methylthio, R² is hydrogen and R³ has the values given in Table 1.

### Table 13

The compounds of Table 13 are of the general formula (I) where Q¹ is ethyl, Q² is hydrogen, Y is oxygen, R¹ is ethyl, R² is hydrogen and R³ has the values given in Table 1.

### Table 14

The compounds of Table 14 are of the general formula (I) where Q¹ is ethyl, Q² is fluoro, Y is oxygen, R¹ is ethyl, R² is hydrogen and R³ has the values given in Table 1.

### Table 15

The compounds of Table 15 are of the general formula (I) where Q¹ is ethyl, Q² is chloro, Y is oxygen, R¹ is ethyl, R² is hydrogen and R³ has the values given in Table 1.

### Table 16

The compounds of Table 16 are of the general formula (I) where Q¹ is ethyl, Q² is methyl, Y is oxygen, R¹ is ethyl, R² is hydrogen and R³ has the values given in Table 1.

### Table 17

The compounds of Table 17 are of the general formula (I) where Q¹ is ethyl, Q² is hydrogen, Y is oxygen, R¹ is methoxy, R² is hydrogen and R³ has the values given in Table 1.

### Table 18

The compounds of Table 18 are of the general formula (I) where Q¹ is ethyl, Q² is fluoro, Y is oxygen, R¹ is methoxy, R² is hydrogen and R³ has the values given in Table 1.

### Table 19

The compounds of Table 19 are of the general formula (I) where Q¹ is ethyl, Q² is chloro, Y is oxygen, R¹ is methoxy, R² is hydrogen and R³ has the values given in Table 1.

### Table 20

The compounds of Table 20 are of the general formula (I) where Q¹ is ethyl, Q² is methyl, Y is oxygen, R¹ is methoxy, R² is hydrogen and R³ has the values given in Table 1.

### Table 21

The compounds of Table 21 are of the general formula (I) where Q¹ is ethyl, Q² is hydrogen, Y is oxygen, R¹ is methylthio, R² is hydrogen and R³ has the values given in Table 1.

### Table 22

The compounds of Table 22 are of the general formula (I) where Q¹ is ethyl, Q² is fluoro, Y is oxygen, R¹ is methylthio, R² is hydrogen and R³ has the values given in Table 1.

### Table 23

The compounds of Table 23 are of the general formula (I) where Q¹ is ethyl, Q² is chloro, Y is oxygen, R¹ is methylthio, R² is hydrogen and R³ has the values given in Table 1.

### Table 24

The compounds of Table 24 are of the general formula (I) where Q¹ is ethyl, Q² is methyl, Y is oxygen, R¹ is methylthio, R² is hydrogen and R³ has the values given in Table 1.

### Table 25

The compounds of Table 25 are of the general formula (I) where Q¹ is vinyl, Q² is hydrogen, Y is oxygen, R¹ is ethyl, R² is hydrogen and R³ has the values given in Table 1.

### Table 26

The compounds of Table 26 are of the general formula (I) where Q¹ is vinyl, Q² is fluoro, Y is oxygen, R¹ is ethyl, R² is hydrogen and R³ has the values given in Table 1.

### Table 27

The compounds of Table 27 are of the general formula (I) where Q¹ is vinyl, Q² is chloro, Y is oxygen, R¹ is ethyl, R² is hydrogen and R³ has the values given in Table 1.

### Table 28

The compounds of Table 28 are of the general formula (I) where Q¹ is vinyl, Q² is methyl, Y is oxygen, R¹ is ethyl, R² is hydrogen and R³ has the values given in Table 1.

### Table 29

The compounds of Table 29 are of the general formula (I) where Q¹ is vinyl, Q² is hydrogen, Y is oxygen, R¹ is methoxy, R² is hydrogen and R³ has the values given in Table 1.

### Table 30

The compounds of Table 30 are of the general formula (I) where Q¹ is vinyl, Q² is fluoro, Y is oxygen, R¹ is methoxy, R² is hydrogen and R³ has the values given in Table 1.

### Table 31

The compounds of Table 31 are of the general formula (I) where Q¹ is vinyl, Q² is chloro, Y is oxygen, R¹ is methoxy, R² is hydrogen and R³ has the values given in Table 1.

### Table 32

The compounds of Table 32 are of the general formula (I) where Q¹ is vinyl, Q² is methyl, Y is oxygen, R¹ is methoxy, R² is hydrogen and R³ has the values given in Table 1.

### Table 33

The compounds of Table 33 are of the general formula (I) where Q¹ is vinyl, Q² is hydrogen, Y is oxygen, R¹ is methylthio, R² is hydrogen and R³ has the values given in Table 1.

### Table 34

The compounds of Table 34 are of the general formula (I) where Q¹ is vinyl, Q² is fluoro, Y is oxygen, R¹ is methylthio, R² is hydrogen and R³ has the values given in Table 1.

### Table 35

The compounds of Table 35 are of the general formula (I) where Q¹ is vinyl, Q² is chloro, Y is oxygen, R¹ is methylthio, R² is hydrogen and R³ has the values given in Table 1.

### Table 36

The compounds of Table 36 are of the general formula (I) where Q¹ is vinyl, Q² is methyl, Y is oxygen, R¹ is methylthio, R² is hydrogen and R³ has the values given in Table 1.

The compounds of formula (I) may be prepared in an analogous manner as outlined in WO09/030467 and WO09/049716 by chemical reactions known in the art.

The compounds of formula (I) are active fungicides and may be used to control one or more of the following pathogens: *Pyricularia oryzae* (*Magnaporthe grisea*) on rice and wheat and other *Pyricularia* spp. on other hosts; *Puccinia triticina* (or *recondita*)*, Puccinia striiformis* and other rusts on wheat, *Puccinia hordei, Puccinia striiformis* and other rusts on barley, and rusts on other hosts (for example turf, rye, coffee, pears, apples, peanuts, sugar beet, vegetables and ornamental plants); Phakopsora pachyrhizi on soybean, *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca* (*Sphaerotheca fuliginea*) on cucurbits (for example cucumber), *Leveillula taurica* on tomatoes, aubergine and green pepper, *Podosphaera leucotricha* on apples and *Uncinula necator* on vines; *Cochliobolus* spp., *Helminthosporium* spp., *Drechslera* spp. (*Pyrenophora* spp.), *Rhynchosporium* spp., *Mycosphaerella graminicola* (*Septoria tritici*) and *Phaeosphaeria nodorum* (*Stagonospora nodorum* or *Septoria nodorum*)*, Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot (G*lomerella cingulata*)*,* black rot or frogeye leaf spot (*Botryosphaeria obtusa*)*,* Brooks fruit spot (*Mycosphaerella pomi*)*,* Cedar apple rust (*Gymnosporangium juniperi-virginianae*)*,* sooty blotch (*Gloeodes pomigena*)*,* flyspeck (*Schizothyrium pomi*) and white rot *(Botryosphaeria dothidea*)) on apples and pears; *Plasmopara viticola* on *vines; ; Plasmopara halstedii* on sunflower; other downy mildews, such as *Bremia lactucae* on lettuce, *Peronospora* spp. on soybeans, tobacco, onions and other hosts, *Pseudoperonospora humuli* on hops *; Peronosclerospora maydis, P. philippinensis* and *P. sorghi* on maize, sorghum and other hosts and *Pseudoperonospora cubensis* on cucurbits; *Pythium* spp. (including *Pythium ultimum*) on cotton, maize, soybean, sugarbeet, vegetables, turf and other hosts; *Phytophthora infestans* on potatoes and tomatoes and other *Phytophthora* spp. on vegetables, strawberries, avocado, pepper, ornamentals, tobacco, cocoa and other hosts; *Aphanomyces* spp. on sugarbeet and other hosts; *Thanatephorus cucumeris* on rice, wheat, cotton, soybean, maize, sugarbeet and turf and other hosts *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Diaporthe* spp. on citrus, soybean, melon, pears, lupin and other hosts; *Elsinoe* spp. on citrus, vines, olives, pecans, roses and other hosts; *Verticillium* spp. on a range of hosts including hops, potatoes and tomatoes; *Pyrenopeziza* spp. on oil-seed rape and other hosts; *Oncobasidium theobromae* on cocoa causing vascular streak dieback; *Fusarium* spp. incl. *Fusarium culmorum, F. graminearum, F. langsethiae, F. moniliforme, F. proliferatum, F. subglutinans, F. solani* and *F. oxysporum* on wheat, barely, rye, oats, maize, cotton, soybean, sugarbeet and other hosts, *Typhula* spp., *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; Thielaviopsis basicola on cotton, vegetables and other hosts; Verticillium spp. on cotton, vegetables and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola, Pseudopeziza tracheiphila* and *Stereum hirsutum;* other pathogens on trees (for example *Lophodermium seditiosum*) or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans;* and fungal vectors of viral diseases (for example *Polymyxa graminis* on cereals as the vector of barley yellow mosaic virus (BYMV) and *Polymyxa betae* on sugar beet as the vector of rhizomania).

Preferably, the following pathogens are controlled: *Pyricularia oryzae (Magnaporthe grisea*) on rice and wheat and other *Pyricularia* spp. on other hosts; *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca* (*Sphaerotheca fuliginea*) on cucurbits (for example cucumber), *Leveillula taurica* on tomatoes, aubergine and green pepper, *Podosphaera leucotricha* on apples and *Uncinula necator* on vines; *Helminthosporium* spp., *Drechslera* spp. (*Pyrenophora* spp.), *Rhynchosporium* spp. *Mycosphaerella graminicola* (*Septoria tritici*) and *Phaeosphaeria nodorum* (*Stagonospora nodorum* or *Septoria nodorum*)*, Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Aspergillus* spp. and *Aureobasidium* spp. on wheat, lumber and other hosts; *Ascochyta* spp. on peas, wheat, barley and other hosts; *Stemphylium* spp. (*Pleospora* spp.) on apples, pears, onions and other hosts; summer diseases (for example bitter rot (*Glomerella cingulata*)*,* black rot or frogeye leaf spot (*Botryosphaeria obtusa*)*,* Brooks fruit spot (*Mycosphaerella pomi*)*,* Cedar apple rust (*Gymnosporangium juniperi-virginianae*)*,* sooty blotch (*Gloeodes pomigena*)*,* flyspeck (*Schizothyrium pomi*) and white rot (*Botryosphaeria dothidea*)) on apples and pears; *Plasmopara viticola* on vines; *Plasmopara halstedii* on sunflower; other downy mildews, such as *Bremia lactucae* on lettuce, *Peronospora* spp. on soybeans, tobacco, onions and other hosts, *Pseudoperonospora humuli* on hops ; *Peronosclerospora maydis, P. philippinensis* and *P. sorghi on* maize, sorghum and other hosts and *Pseudoperonospora cubensis* on cucurbits; *Pythium* spp. (including *Pythium ultimum*) on cotton, maize, soybean, sugarbeet, vegetables, turf and other hosts; *Phytophthora infestans* on potatoes and tomatoes and other *Phytophthora* spp. on vegetables, strawberries, avocado, pepper, ornamentals, tobacco, cocoa and other hosts; *Aphanomyces* spp. on sugarbeet and other hosts; *Thanatephorus cucumeris* on rice, wheat, cotton, soybean, maize, sugarbeet and turf and other hosts *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Fusarium* spp. incl. *Fusarium culmorum, F. graminearum, F. langsethiae, F. moniliforme, F. proliferatum, F. subglutinans, F. solani* and *F. oxysporum* on wheat, barely, rye, oats, maize, cotton, soybean, sugarbeet and other hosts, *Microdochium nivale, Ustilago* spp., *Urocystis* spp., *Tilletia* spp. and *Claviceps purpurea* on a variety of hosts but particularly wheat, barley, turf and maize; *Ramularia* spp. on sugar beet, barley and other hosts; Thielaviopsis basicola on cotton, vegetables and other hosts; Verticillium spp. on cotton, vegetables and other hosts; post-harvest diseases particularly of fruit (for example *Penicillium digitatum, Penicillium italicum* and *Trichoderma viride* on oranges, *Colletotrichum musae* and *Gloeosporium musarum* on bananas and *Botrytis cinerea* on grapes); other pathogens on vines, notably *Eutypa lata, Guignardia bidwellii, Phellinus igniarus, Phomopsis viticola, Pseudopeziza tracheiphila* and *Stereum hirsutum;* other pathogens on trees (for example *Lophodermium seditiosum*) or lumber, notably *Cephaloascus fragrans, Ceratocystis* spp., *Ophiostoma piceae, Penicillium* spp., *Trichoderma pseudokoningii, Trichoderma viride, Trichoderma harzianum, Aspergillus niger, Leptographium lindbergi* and *Aureobasidium pullulans.*

More preferably, the following pathogens are controlled: *Pyricularia oryzae (Magnaporthe grisea*) on rice and wheat and other *Pyricularia* spp. on other hosts; *Erysiphe cichoracearum* on cucurbits (for example melon); *Blumeria* (or *Erysiphe*) *graminis* (powdery mildew) on barley, wheat, rye and turf and other powdery mildews on various hosts, such as *Sphaerotheca macularis* on hops, *Sphaerotheca fusca* (*Sphaerotheca fuliginea*) on cucurbits (for example cucumber), *Leveillula taurica* on tomatoes, aubergine and green pepper, *Podosphaera leucotricha* on apples and *Uncinula necator* on vines; *Mycosphaerella graminicola (Septoria tritici)* and *Phaeosphaeria nodorum* (*Stagonospora nodorum* or *Septoria nodorum*)*, Pseudocercosporella herpotrichoides* and *Gaeumannomyces graminis* on cereals (for example wheat, barley, rye), turf and other hosts; *Cercospora arachidicola* and *Cercosporidium personatum* on peanuts and other *Cercospora* spp. on other hosts, for example sugar beet, bananas, soya beans and rice; *Botrytis cinerea* (grey mould) on tomatoes, strawberries, vegetables, vines and other hosts and other *Botrytis* spp. on other hosts; *Alternaria* spp. on vegetables (for example carrots), oil-seed rape, apples, tomatoes, potatoes, cereals (for example wheat) and other hosts; *Venturia* spp. (including *Venturia inaequalis* (scab)) on apples, pears, stone fruit, tree nuts and other hosts; *Cladosporium* spp. on a range of hosts including cereals (for example wheat) and tomatoes; *Monilinia* spp. on stone fruit, tree nuts and other hosts; *Didymella* spp. on tomatoes, turf, wheat, cucurbits and other hosts; *Phoma* spp. on oil-seed rape, turf, rice, potatoes, wheat and other hosts; *Plasmopara viticola* on vines; ; *Plasmopara halstedii* on sunflower; other downy mildews, such as *Bremia lactucae* on lettuce, *Peronospora* spp. on soybeans, tobacco, onions and other hosts, *Pseudoperonospora humuli* on hops ; *Peronosclerospora maydis, P. philippinensis* and *P. sorghi* on maize, sorghum and other hosts and *Pseudoperonospora cubensis* on cucurbits; *Pythium* spp. (including *Pythium ultimum)* on cotton, maize, soybean, sugarbeet, vegetables, turf and other hosts; *Phytophthora infestans* on potatoes and tomatoes and other *Phytophthora* spp. on vegetables, strawberries, avocado, pepper, ornamentals, tobacco, cocoa and other hosts; *Aphanomyces* spp. on sugarbeet and other hosts; *Thanatephorus cucumeris* on rice, wheat, cotton, soybean, maize, sugarbeet and turf and other hosts *Rhizoctonia* spp. on various hosts such as wheat and barley, peanuts, vegetables, cotton and turf; *Sclerotinia* spp. on turf, peanuts, potatoes, oil-seed rape and other hosts; *Sclerotium* spp. on turf, peanuts and other hosts; *Gibberella fujikuroi* on rice; *Colletotrichum* spp. on a range of hosts including turf, coffee and vegetables; *Laetisaria fuciformis* on turf; *Mycosphaerella* spp. on bananas, peanuts, citrus, pecans, papaya and other hosts; *Fusarium* spp. incl. *Fusarium culmorum, F. graminearum, F. langsethiae, F. moniliforme, F. proliferatum, F. subglutinans, F. solani* and *F. oxysporum* on wheat, barely, rye, oats, maize, cotton, soybean, sugarbeet and other hosts; and *Microdochium nivale.*

A compound of formula (I) may move acropetally, basipetally or locally in plant tissue to be active against one or more fungi. Moreover, a compound of formula (I) may be volatile enough to be active in the vapour phase against one or more fungi on the plant.

The invention therefore provides a method of combating or controlling phytopathogenic fungi which comprises applying a fungicidally effective amount of a compound of formula (I), or a composition containing a compound of formula (I), to a plant, to a seed of a plant, to the locus of the plant or seed or to soil or any other plant growth medium, e.g. nutrient solution.

The term "plant" as used herein includes seedlings, bushes and trees. Furthermore, the fungicidal method of the invention includes protectant, curative, systemic, eradicant and antisporulant treatments.

The term "plant" as used herein also includes crops of useful plants in which the compositions according to the invention can be used and includes especially cereals, in particular wheat and barley, rice, corn, rape, sugarbeet, sugarcane, soybean, cotton, sunflower, peanut and plantation crops.

The term "crops" is to be understood as also including crops that have been rendered tolerant to herbicides or classes of herbicides (for example ALS, GS, EPSPS, PPO and HPPD inhibitors) as a result of conventional methods of breeding or genetic engineering.

The compounds of formula (I) are preferably used for agricultural, horticultural and turfgrass purposes in the form of a composition.

In order to apply a compound of formula (I) to a plant, to a seed of a plant, to the locus of the plant or seed or to soil or any other growth medium, a compound of formula (I) is usually formulated into a composition which includes, in addition to the compound of formula (I), a suitable inert diluent or carrier and, optionally, a surface active agent (SFA). SFAs are chemicals that are able to modify the properties of an interface (for example, liquid/solid, liquid/air or liquid/liquid interfaces) by lowering the interfacial tension and thereby leading to changes in other properties (for example dispersion, emulsification and wetting). It is preferred that all compositions (both solid and liquid formulations) comprise, by weight, 0.0001 to 95%, more preferably 1 to 85%, for example 5 to 60%, of a compound of formula (I). The composition is generally used for the control of fungi such that a compound of formula (I) is applied at a rate of from 0.1 g to 10kg per hectare, preferably from 1 g to 6kg per hectare, more preferably from 1 g to 1 kg per hectare.

When used in a seed dressing, a compound of formula (I) is used at a rate of 0.0001g to 1 Og (for example 0.001 g or 0.05g), preferably 0.005g to 10g, more preferably 0.005g to 4g, per kilogram of seed.

In another aspect the present invention provides a fungicidal composition comprising a fungicidally effective amount of a compound of formula (I) and a suitable carrier or diluent therefor.

In a still further aspect the invention provides a method of combating and controlling fungi at a locus, which comprises treating the fungi, or the locus of the fungi with a fungicidally effective amount of a composition comprising a compound of formula (I). The compositions can be chosen from a number of formulation types, including dustable powders (DP), soluble powders (SP), water soluble granules (SG), water dispersible granules (WG), wettable powders (WP), granules (GR) (slow or fast release), soluble concentrates (SL), oil miscible liquids (OL), ultra low volume liquids (UL), emulsifiable concentrates (EC), dispersible concentrates (DC), emulsions (both oil in water (EW) and water in oil (EO)), micro-emulsions (ME), suspension concentrates (SC), aerosols, fogging/smoke formulations, capsule suspensions (CS) and seed treatment formulations. The formulation type chosen in any instance will depend upon the particular purpose envisaged and the physical, chemical and biological properties of the compound of formula (I).

Dustable powders (DP) may be prepared by mixing a compound of formula (I) with one or more solid diluents (for example natural clays, kaolin, pyrophyllite, bentonite, alumina, montmorillonite, kieselguhr, chalk, diatomaceous earths, calcium phosphates, calcium and magnesium carbonates, sulphur, lime, flours, talc and other organic and inorganic solid carriers) and mechanically grinding the mixture to a fine powder.

Soluble powders (SP) may be prepared by mixing a compound of formula (I) with one or more water-soluble inorganic salts (such as sodium bicarbonate, sodium carbonate or magnesium sulphate) or one or more water-soluble organic solids (such as a polysaccharide) and, optionally, one or more wetting agents, one or more dispersing agents or a mixture of said agents to improve water dispersibility/solubility. The mixture is then ground to a fine powder. Similar compositions may also be granulated to form water soluble granules (SG).

Wettable powders (WP) may be prepared by mixing a compound of formula (I) with one or more solid diluents or carriers, one or more wetting agents and, preferably, one or more dispersing agents and, optionally, one or more suspending agents to facilitate the dispersion in liquids. The mixture is then ground to a fine powder. Similar compositions may also be granulated to form water dispersible granules (WG).

Granules (GR) may be formed either by granulating a mixture of a compound of formula (I) and one or more powdered solid diluents or carriers, or from pre-formed blank granules by absorbing a compound of formula (I) (or a solution thereof, in a suitable agent) in a porous granular material (such as pumice, attapulgite clays, fuller's earth, kieselguhr, diatomaceous earths or ground corn cobs) or by adsorbing a compound of formula (I) (or a solution thereof, in a suitable agent) on to a hard core material (such as sands, silicates, mineral carbonates, sulphates or phosphates) and drying if necessary. Agents which are commonly used to aid absorption or adsorption include solvents (such as aliphatic and aromatic petroleum solvents, alcohols, ethers, ketones and esters) and sticking agents (such as polyvinyl acetates, polyvinyl alcohols, dextrins, sugars and vegetable oils). One or more other additives may also be included in granules (for example an emulsifying agent, wetting agent or dispersing agent).

Dispersible Concentrates (DC) may be prepared by dissolving a compound of formula (I) in water or an organic solvent, such as a ketone, alcohol or glycol ether. These solutions may contain a surface active agent (for example to improve water dilution or prevent crystallisation in a spray tank).

Emulsifiable concentrates (EC) or oil-in-water emulsions (EW) may be prepared by dissolving a compound of formula (I) in an organic solvent (optionally containing one or more wetting agents, one or more emulsifying agents or a mixture of said agents). Suitable organic solvents for use in ECs include aromatic hydrocarbons (such as alkylbenzenes or alkylnaphthalenes, exemplified by SOLVESSO 100, SOLVESSO 150 and SOLVESSO 200; SOLVESSO is a Registered Trade Mark), ketones (such as cyclohexanone or methylcyclohexanone), alcohols (such as benzyl alcohol, furfuryl alcohol or butanol), N-alkylpyrrolidones (such as N-methylpyrrolidone or N-octyl-pyrrolidone), dimethyl amides of fatty acids (such as C₈-C₁₀ fatty acid dimethylamide) and chlorinated hydrocarbons. An EC product may spontaneously emulsify on addition to water, to produce an emulsion with sufficient stability to allow spray application through appropriate equipment. Preparation of an EW involves obtaining a compound of formula (I) either as a liquid (if it is not a liquid at ambient temperature, it may be melted at a reasonable temperature, typically below 70°C) or in solution (by dissolving it in an appropriate solvent) and then emulsifying the resultant liquid or solution into water containing one or more SFAs, under high shear, to produce an emulsion. Suitable solvents for use in EWs include vegetable oils, chlorinated hydrocarbons (such as chlorobenzenes), aromatic solvents (such as alkylbenzenes or alkylnaphthalenes) and other appropriate organic solvents that have a low solubility in water.

Microemulsions (ME) may be prepared by mixing water with a blend of one or more solvents with one or more SFAs, to produce spontaneously a thermodynamically stable isotropic liquid formulation. A compound of formula (I) is present initially in either the water or the solvent/SFA blend. Suitable solvents for use in MEs include those hereinbefore described for use in ECs or in EWs. An ME may be either an oil-in-water or a water-in-oil system (which system is present may be determined by conductivity measurements) and may be suitable for mixing water-soluble and oil-soluble pesticides in the same formulation. An ME is suitable for dilution into water, either remaining as a microemulsion or forming a conventional oil-in-water emulsion.

Suspension concentrates (SC) may comprise aqueous or non-aqueous suspensions of finely divided insoluble solid particles of a compound of formula (I). SCs may be prepared by ball or bead milling the solid compound of formula (I) in a suitable medium, optionally with one or more dispersing agents, to produce a fine particle suspension of the compound. One or more wetting agents may be included in the composition and a suspending agent may be included to reduce the rate at which the particles settle. Alternatively, a compound of formula (I) may be dry milled and added to water, containing agents hereinbefore described, to produce the desired end product.

Aerosol formulations comprise a compound of formula (I) and a suitable propellant (for example n-butane). A compound of formula (I) may also be dissolved or dispersed in a suitable medium (for example water or a water miscible liquid, such as n-propanol) to provide compositions for use in non-pressurised, hand-actuated spray pumps.

A compound of formula (I) may be mixed in the dry state with a pyrotechnic mixture to form a composition suitable for generating, in an enclosed space, a smoke containing the compound.

Capsule suspensions (CS) may be prepared in a manner similar to the preparation of EW formulations but with an additional polymerisation stage such that an aqueous dispersion of oil droplets is obtained, in which each oil droplet is encapsulated by a polymeric shell and contains a compound of formula (I) and, optionally, a carrier or diluent therefor. The polymeric shell may be produced by either an interfacial polycondensation reaction or by a coacervation procedure. The compositions may provide for controlled release of the compound of formula (I) and they may be used for seed treatment. A compound of formula (I) may also be formulated in a biodegradable polymeric matrix to provide a slow, controlled release of the compound.

A composition may include one or more additives to improve the biological performance of the composition (for example by improving wetting, retention or distribution on surfaces; resistance to rain on treated surfaces; or uptake or mobility of a compound of formula (I)). Such additives include surface active agents, spray additives based on oils, for example certain mineral oils or natural plant oils (such as soy bean and rape seed oil), and blends of these with other bio-enhancing adjuvants (ingredients which may aid or modify the action of a compound of formula (I)).

A compound of formula (I) may also be formulated for use as a seed treatment, for example as a powder composition, including a powder for dry seed treatment (DS), a water soluble powder (SS) or a water dispersible powder for slurry treatment (WS), or as a liquid composition, including a flowable concentrate (FS), a solution (LS) or a capsule suspension (CS). The preparations of DS, SS, WS, FS and LS compositions are very similar to those of, respectively, DP, SP, WP, SC and DC compositions described above. Compositions for treating seed may include an agent for assisting the adhesion of the composition to the seed (for example a mineral oil or a film-forming barrier). Wetting agents, dispersing agents and emulsifying agents may be SFAs of the cationic, anionic, amphoteric or non-ionic type.

Suitable SFAs of the cationic type include quaternary ammonium compounds (for example cetyltrimethyl ammonium bromide), imidazolines and amine salts. Suitable anionic SFAs include alkali metals salts of fatty acids, salts of aliphatic monoesters of sulphuric acid (for example sodium lauryl sulphate), salts of sulphonated aromatic compounds (for example sodium dodecylbenzenesulphonate, calcium dodecylbenzenesulphonate, butyl naphthalene sulphonate and mixtures of sodium diisopropyl- and tri-isopropyl-naphthalene sulphonates), ether sulphates, alcohol ether sulphates (for example sodium laureth-3-sulphate), ether carboxylates (for example sodium laureth-3-carboxylate), phosphate esters (products from the reaction between one or more fatty alcohols and phosphoric acid (predominately mono-esters) or phosphorus pentoxide (predominately di-esters), for example the reaction between lauryl alcohol and tetraphosphoric acid; additionally these products may be ethoxylated), sulphosuccinamates, paraffin or olefin sulphonates, taurates and lignosulphonates. Suitable SFAs of the amphoteric type include betaines, propionates and glycinates. Suitable SFAs of the non-ionic type include condensation products of alkylene oxides, such as ethylene oxide, propylene oxide, butylene oxide or mixtures thereof, with fatty alcohols (such as oleyl alcohol or cetyl alcohol) or with alkylphenols (such as octylphenol, nonylphenol or octylcresol); partial esters derived from long chain fatty acids or hexitol anhydrides; condensation products of said partial esters with ethylene oxide; block polymers (comprising ethylene oxide and propylene oxide); alkanolamides; simple esters (for example fatty acid polyethylene glycol esters); amine oxides (for example lauryl dimethyl amine oxide); and lecithins.

Suitable suspending agents include hydrophilic colloids (such as polysaccharides, polyvinylpyrrolidone or sodium carboxymethylcellulose) and swelling clays (such as bentonite or attapulgite).

A compound of formula (I) may be applied by any of the known means of applying fungicidal compounds. For example, it may be applied, formulated or unformulated, to any part of the plant, including the foliage, stems, branches or roots, to the seed before it is planted or to other media in which plants are growing or are to be planted (such as soil surrounding the roots, the soil generally, paddy water or hydroponic culture systems), directly or it may be sprayed on, dusted on, applied by dipping, applied as a cream or paste formulation, applied as a vapour or applied through distribution or incorporation of a composition (such as a granular composition or a composition packed in a water-soluble bag) in soil or an aqueous environment.

A compound of formula (I) may also be injected into plants or sprayed onto vegetation using electrodynamic spraying techniques or other low volume methods, or applied by land or aerial irrigation systems.

Compositions for use as aqueous preparations (aqueous solutions or dispersions) are generally supplied in the form of a concentrate containing a high proportion of the active ingredient, the concentrate being added to water before use. These concentrates, which may include DCs, SCs, ECs, EWs, MEs, SGs, SPs, WPs, WGs and CSs, are often required to withstand storage for prolonged periods and, after such storage, to be capable of addition to water to form aqueous preparations which remain homogeneous for a sufficient time to enable them to be applied by conventional spray equipment. Such aqueous preparations may contain varying amounts of a compound of formula (I) (for example 0.0001 to 10%, by weight) depending upon the purpose for which they are to be used.

A compound of formula (I) may be used in mixtures with fertilisers (for example nitrogen-, potassium- or phosphorus-containing fertilisers). Suitable formulation types include granules of fertiliser. The mixtures suitably contain up to 25% by weight of the compound of formula (I).

The invention therefore also provides a fertiliser composition comprising a fertiliser and a compound of formula (I).
The compositions of this invention may contain other compounds having biological activity, for example micronutrients or compounds having similar or complementary fungicidal activity or which possess plant growth regulating, herbicidal, insecticidal, nematicidal or acaricidal activity.

By including another fungicide, the resulting composition may have a broader spectrum of activity or a greater level of intrinsic activity than the compound of formula (I) alone. Further, the other fungicide may have a synergistic effect on the fungicidal activity of the compound of formula (I).

The compound of formula (I) may be the sole active ingredient of the composition or it may be admixed with one or more additional active ingredients such as a pesticide, fungicide, synergist, herbicide or plant growth regulator where appropriate. An additional active ingredient may: provide a composition having a broader spectrum of activity or increased persistence at a locus; synergise the activity or complement the activity (for example by increasing the speed of effect or overcoming repellency) of the compound of formula (I); or help to overcome or prevent the development of resistance to individual components. The particular additional active ingredient will depend upon the intended utility of the composition.

Examples of further fungicidal compounds which may be included in the composition of the invention are AC 382042 (N-(1-cyano-1,2-dimethylpropyl)-2-(2,4-dichlorophenoxy) propionamide), acibenzolar-S-methyl, alanycarb, aldimorph, anilazine, azaconazole, azafenidin, azoxystrobin, benalaxyl, benomyl, benthiavalicarb, biloxazol, bitertanol, blasticidin S, boscalid (new name for nicobifen), bromuconazole, bupirimate, captafol, captan, carbendazim, carbendazim chlorhydrate, carboxin, carpropamid, carvone, CGA 41396, CGA 41397, chinomethionate, chlorbenzthiazone, chlorothalonil, chlorozolinate, clozylacon, copper containing compounds such as copper oxychloride, copper oxyquinolate, copper sulphate, copper tallate, and Bordeaux mixture, cyamidazosulfamid, cyazofamid (IKF-916), cyflufenamid, cymoxanil, cyproconazole, cyprodinil, debacarb, di-2-pyridyl disulphide 1,1'-dioxide, dichlofluanid, diclocymet, diclomezine, dicloran, diethofencarb, difenoconazole, difenzoquat, diflumetorim, O,O-di-iso-propyl-S-benzyl thiophosphate, dimefluazole, dimetconazole, dimethirimol, dimethomorph, dimoxystrobin, diniconazole, dinocap, dithianon, dodecyl dimethyl ammonium chloride, dodemorph, dodine, doguadine, edifenphos, epoxiconazole, ethaboxam, ethirimol, ethyl (Z)-N-benzyl-N([methyl(methyl-thioethylideneaminooxy-carbonyl)amino]thio)-β-alaninate, etridiazole, famoxadone, fenamidone, fenarimol, fenbuconazole, fenfuram, fenhexamid, fenoxanil (AC 382042), fenpiclonil, fenpropidin, fenpropimorph, fentin acetate, fentin hydroxide, ferbam, ferimzone, fluazinam, fludioxonil, flumetover, flumorph, fluoroimide, fluoxastrobin, fluquinconazole, flusilazole, flusulfamide, flutolanil, flutriafol, folpet, fosetyl-aluminium, fuberidazole, furalaxyl, furametpyr, guazatine, hexaconazole, hydroxyisoxazole, hymexazole, imazalil, imibenconazole, iminoctadine, iminoctadine triacetate, ipconazole, iprobenfos, iprodione, iprovalicarb, isopropanyl butyl carbamate, isoprothiolane, kasugamycin, kresoxim-methyl, LY186054, LY211795, LY 248908, mancozeb, maneb, mefenoxam, mepanipyrim, mepronil, metalaxyl, metalaxyl M, metconazole, metiram, metiram-zinc, metominostrobin, metrafenone, MON65500 (N-allyl-4,5-dimethyl-2-trimethylsilylthiophene-3-carboxamide), myclobutanil, NTN0301, neoasozin, nickel dimethyldithiocarbamate, nitrothale-isopropyl, nuarimol, ofurace, organomercury compounds, orysastrobin, oxadixyl, oxasulfuron, oxolinic acid, oxpoconazole, oxycarboxin, pefurazoate, penconazole, pencycuron, phenazin oxide, phosphorus acids, phthalide, picoxystrobin, polyoxin D, polyram, probenazole, prochloraz, procymidone, propamocarb, propamocarb hydrochloride, propiconazole, propineb, propionic acid, proquinazid, prothioconazole, pyraclostrobin, pyrazophos, pyrifenox, pyrimethanil, pyroquilon, pyroxyfur, pyrrolnitrin, quaternary ammonium compounds, quinomethionate, quinoxyfen, quintozene, silthiofam (MON 65500), S-imazalil, simeconazole, sipconazole, sodium pentachlorophenate, spiroxamine, streptomycin, sulphur, tebuconazole, tecloftalam, tecnazene, tetraconazole, thiabendazole, thifluzamide, 2-(thiocyano-methylthio)benzothiazole, thiophanate-methyl, thiram, tiadinil, timibenconazole, tolclofos-methyl, tolylfluanid, triadimefon, triadimenol, triazbutil, triazoxide, tricyclazole, tridemorph, trifloxystrobin, triflumizole, triforine, triticonazole, validamycin A, vapam, vinclozolin, XRD-563, zineb, ziram, zoxamide and the compounds of the formulae:

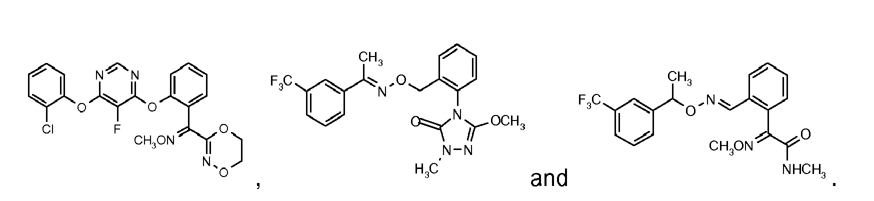

The compounds of formula (I) may be mixed with soil, peat or other rooting media for the protection of plants against seed-borne, soil-borne or foliar fungal diseases.

Some mixtures may comprise active ingredients, which have significantly different physical, chemical or biological properties such that they do not easily lend themselves to the same conventional formulation type. In these circumstances other formulation types may be prepared. For example, where one active ingredient is a water insoluble solid and the other a water insoluble liquid, it may nevertheless be possible to disperse each active ingredient in the same continuous aqueous phase by dispersing the solid active ingredient as a suspension (using a preparation analogous to that of an SC) but dispersing the liquid active ingredient as an emulsion (using a preparation analogous to that of an EW). The resultant composition is a suspoemulsion (SE) formulation.

The invention is illustrated by the following Examples in which the following abbreviations are used:
- ml =: millilitres
- g =: grammes
- ppm =: parts per million
- M⁺ =: mass ion
- s =: singlet
- d =: doublet
- br s =: broad singlet
- t =: triplet

- DMF ₌: dimethylformamide
- NMR ₌: nuclear magnetic resonance
- HPLC =: high performance liquid chromatography
- q =: quartet
- m =: multiplet
- ppm =: parts per million

### Example 1

Compound 1 was obtained following published procedures, for example those mentioned in WO09/030467. Compound 2 was prepared according to sequence 1 depicted below.

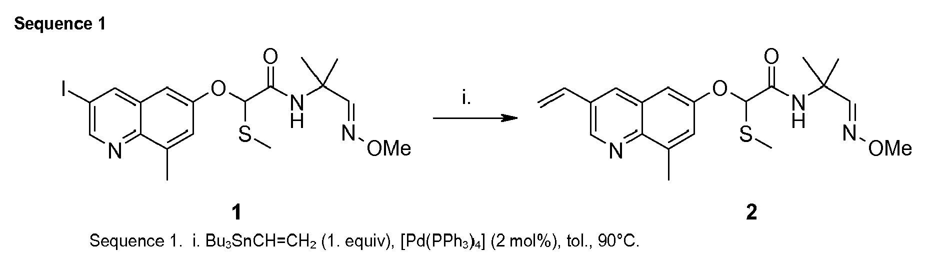

### Step 1: 2

A solution of **1** (180 mg, 0.37 mmol) and tributyl(vinyl)tin (110 mL, 0.37 mmol, 1 equiv) in toluene (6 mL) was sparged with argon gas for 10 min. To this mixture was added [Pd(PPh₃)₄] (9 mg, 2 mol%) and the reaction was heated to 90°C and stirred for 16 h under an atmosphere of argon. The reaction was then cooled to rt and quenched by addition of a solution of saturated Na₂C0₃ (10 mL) and the resulting mixture was stirred for an additional 4 h. The mixture was extracted twice with EtOAc, the combined organic phase was sequentialy washed with ammonia (5% solution in water) and brine, dried over Na₂SO₄, filtered and evaporated. The crude product was purified by column chromatography over SiO₂ (EtOAc/cyclohexane 1:2) to yield **2** (115 mg, 80%) as a white solid.
¹H NMR (CDCl₃) δ ppm: 8.92 (1 H, d), 7.98 (1 H, d), 7.60 (1 H,s), 7.41 (1 H, s), 7.19 (1 H, d), 6.86 (1 H, dd), 5.98 (1 H, d), 5.65 (1 H, s), 5.45 (1 H, d), 3.90 (3H, s), 2.80 (3H, s), 2.21 (3H, s), 1.61 (3H, s), 1.59 (3H, s). m.p = 87-89°C

### Example 2

Compound **3** was obtained following published procedures. Compound 7 was prepared according to sequence 2 depicted below.

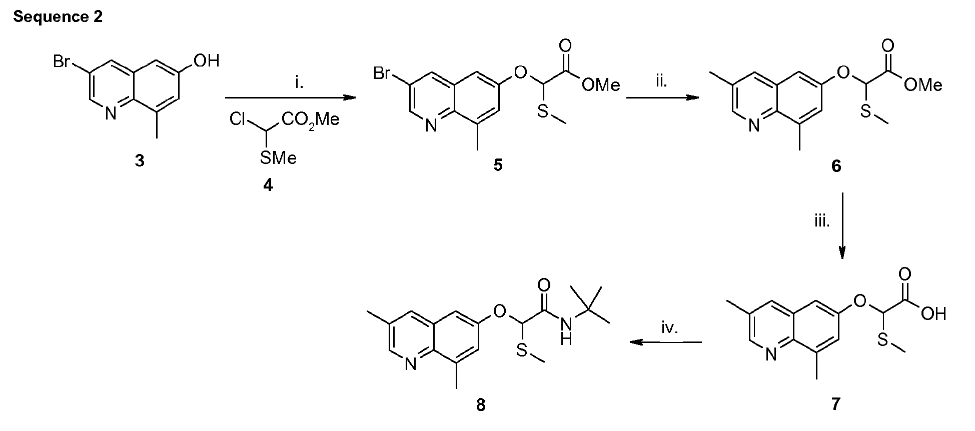

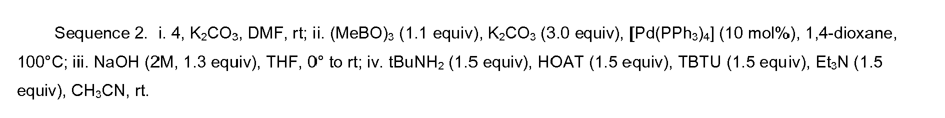

### Step 1: 5

To a solution of **3** (83.35 g, 350.09 mmol) in DMF (700 mL) at room temperature was added grinded K₂C0₃ (145.2 g, 1.05 mol, 3 equiv) followed by **4** (64.95 g, 420.10 mmol, 1.2 equiv). The resulting brown suspension was heated to 60°C and stirred for 5h at this temperature. The reaction mixture was poured in water (3 L) and extracted with EtOAc (5x800 mL). The combined organic layers were dried on MgSO₄, filtered and evaporated. The residue was purified by column chromatography (Heptane/EtOAc, 8:2) to yield crude **5** (100 g) as orange crystal which was further purified by crystallization from EtOAc to yield pure **5** (73.88 g, 59%) of as a yellow crystals.
¹H NMR (CDCl₃) δ ppm: 8.80 (1 H, d), 8.40 (1 H, s), 7.36 (1 H,d), 6.97 (1 H, dd), 5.77 (1 H, s), 3.89 (3H, s), 2.76 (3H, s), 2.23 (3H, s).

### Step 2: 6

A solution of **5** (4.00 g, 11.23 mmol) and trimethylboroxine (1.72 mL, 12.35 mmol, 1.1 equiv) in 1,4-dioxane (90 mL) was purged with argon gas for 10 min. To this mixture was added [Pd(PPh₃)₄] (1.3 g, 10 mol%) and K₂COₛ (4.66 g, 33.69 mmol, 3.0 equiv) and the reaction was heated to 100°C and stirred for 5 h under an atmosphere of argon. The reaction was then cooled to rt and diluted with EtOAc (500 mL), washed with water and brine, dried over Na₂S0₄, filtered and evaporated. The crude product was purified by column chromatography over Si0₂ (EtOAc/cyclohexane 1:3) to yield **6** (1.67 g, 51 %) as a light-yellow oil.
¹H NMR (CDCl₃) 8 ppm: 8.68 (1 H, d), 7.80 (1 H, s), 7.30 (1 H,d), 7.00 (1 H, dd), 5.65 (1 H, s), 3.90 (3H, s), 2.78 (3H, s), 2.50 (3H, s), 2.25 (3H, s).

### Step 3: 7

To a solution of ester **6** (1.67 g, 5.70 mmol) in THF (25 mL) at 0°C was added NaOH (1 M, 7.5 mL, 1.3 equiv) dropwise over 5 min, after completion of the addition the reaction was brought back to rt and stirred for 1 h. The reaction mixture was brought to pH 2 by slow addition of HCI (2M). The resulting mixture was extracted four times with EtOAc, dried over Na₂S0₄, filtered and evaporated to yield acid 7 (1.39 g 87%) a light-yellow solid.
¹ H NMR (*d₆*-dmso) 8 ppm: 13.45 (1 H, bs), 8.64 (1 H, d), 7.95 (1 H, d), 7.32 (1 H, d), 7.21
(1 H, dd), 6.03 (1 H, s), 2.66 (3H, s), 2.48 (3H, s), 2.15 (3H, s).

### Step 4: 8

To solution of **7** (150 mg, 0.54 mmol) in acetonitrile (10 mL) was added sequentially Et₃N (0.260 mL, 3.5 equiv), HOAT (110 mg, 1.5 equiv), tert-butylamine (85 mL, 1.5 equiv) and TBTU (260 mg, 1.5 equiv). The reaction was then stirred for 2 h at rt. The reaction mixture was poured into a saturated NaHC0₃ solution and extracted with EtOAc. The combined organic phase were washed with brine, dried over Na₂SO₄, filtered and evaporated. The crude product was purified by column chromatography over Si0₂ (EtOAc/cyclohexane 1:2) to yield amide **8** (117 mg, 65%) as a white solid.
¹ H NMR (CDCl₃) 8 ppm: 8.79 (1 H, d), 7.82 (1 H, s), 7.24 (1 H,d), 7.02 (1 H, dd), 6.50 (1 H, bs), 5.58 (1 H, s), 2.78 (3H, s), 2.51 (3H, s), 2.20 (3H, s), 1.42 (9H, s). m.p = 126-127°C

**Table 37 This table gives analytical data (melting point) for compounds of Tables 1 - 36**

| Compound No. | Structural formula | Compound | m.p. (°C) |
|---|---|---|---|
| 1 | 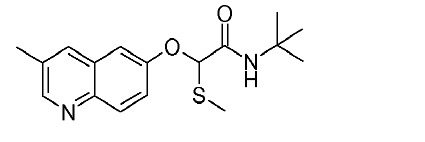 | N-tert-Butyl-2-(3-methyl-quinolin-6 -yloxy)-2-methylsulfanyl-acetamide | 126 - 127 |
| 2 | 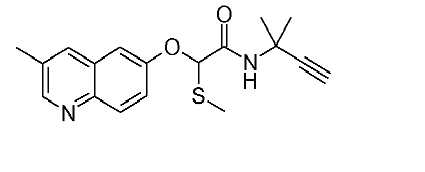 | N-(1,1-Dimethyl-prop-2-ynyl)-2-(3-methyl-quinolin-6-yloxy)-2-methylsulfanyl-acetamide | 132 - 134 |
| 3 | 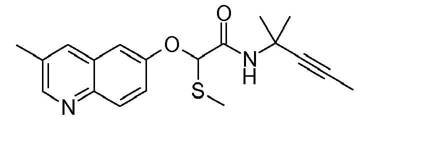 | N-(1,1-Dimethyl-but-2-ynyl)-2-(3-methyl-quinolin-6-yloxy)-2-methylsulfanyl-acetamide | 128 - 129 |
| 4 | 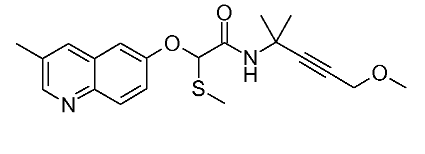 | N-(4-Methoxy-1,1-dimethyl-but-2-ynyl)-2-(3-methyl-quinolin-6-yloxy)-2-methylsulfanyl-acetamide | 100 - 102 |
| 5 | 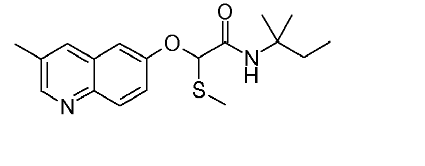 | N-(1,1-Dimethyl-propyl)-2-(3-methyl -quinolin-6-yloxy)-2-methylsulfanyl -acetamide | 103 - 104 |
| 6 | 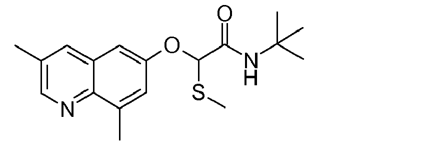 | N-tert-Butyl-2-(3,8-dimethyl-quinolin-6-yloxy)-2-methylsulfanyl-acetamide | 126 - 127 |
| 7 | 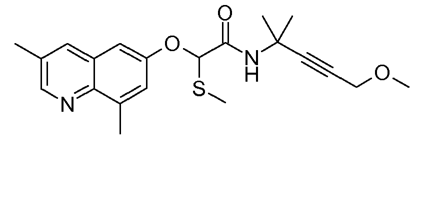 | 2-(3,8-Dimethyl-quinolin-6-yloxy)-N-(4-methoxy-1,1-dimethyl-but-2-ynyl )-2-methylsulfanyl-acetamide | 124 - 126 |
| 8 | 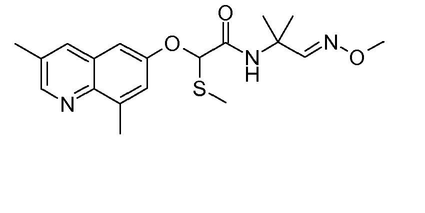 | 2-(3,8-Dimethyl-quinolin-6-yloxy)-N-(2-methoxyimino-1,1-dimethyl-ethyl )-2-methylsulfanyl-acetamide | 109-111 |
| 9 | 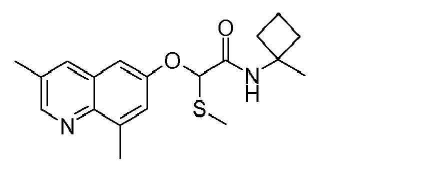 | 2-(3,8-Dimethyl-quinolin-6-yloxy)-N-(1-methyl-cyclobutyl)-2-methylsulf anyl-acetamide | 150-151 |
| 10 | 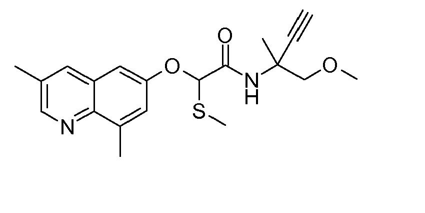 | 2-(3,8-Dimethyl-quinolin-6-yloxy)-N-(1-methoxymethyl-1-methyl-prop-2-y nyl)-2-methylsulfanyl-acetamide | 119 - 120 |
| 11 | 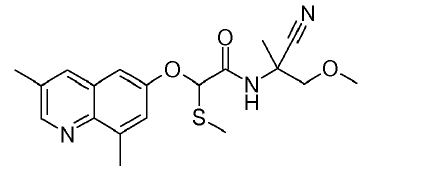 | N-(1-Cyano-2-methoxy-1-methyl-ethyl )-2-(3,8-dimethyl-quinolin-6-yloxy) -2-methylsulfanyl-acetamide | 115 - 117 |
| 12 | 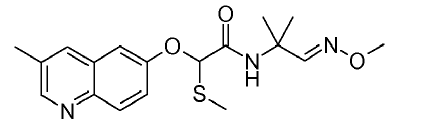 | N-(2-Methoxyimino-1,1-dimethyl-ethy I)-2-(3-methyl-quinolin-6-yloxy)-2-methylsulfanyl-acetamide | 130 - 132 |
| 13 | 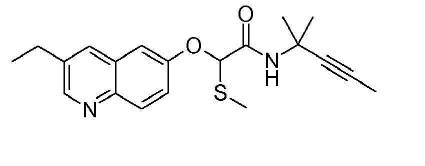 | N-(1,1-Dimethyl-but-2-ynyl)-2-(3-et hyl-quinolin-6-yloxy)-2-methylsulfanyl-acetamide | 133 - 134 |
| 14 | 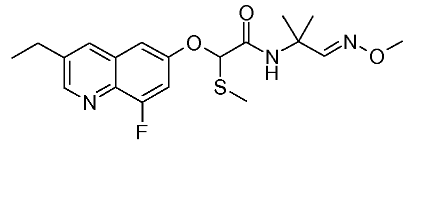 | 2-(3-Ethyl-8-fluoro-quinolin-6-yloxy)-N-(2-methoxyimino-1,1-dimethyl-e thyl)-2-methylsulfanyl-acetamide | 101 - 103 |
| 15 | 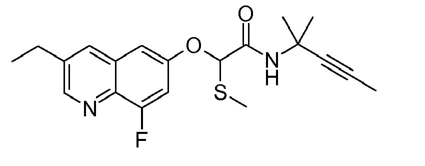 | N-(1,1-Dimethyl-but-2-ynyl)-2-(3-et hyl-8-fluoro-quinolin-6-yloxy)-2-methylsulfanyl-acetamide | 151 - 153 |
| 16 | 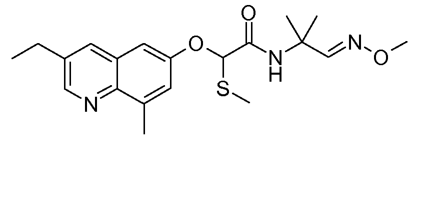 | 2-(3-Ethyl-8-methyl-quinolin-6-yloxy)-N-(2-methoxyimino-1,1-dimethyl-e thyl)-2-methylsulfanyl-acetamide | 105 - 106 |
| 17 | 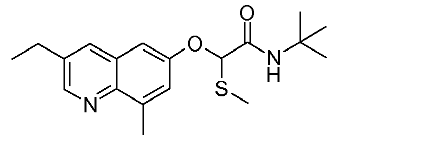 | N-tert-Butyl-2-(3-ethyl-8-methyl-qu inolin-6-yloxy)-2-methylsulfanyl-ac etamide | 153 - 154 |
| 18 | 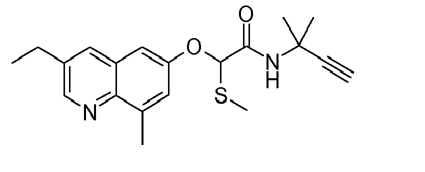 | N-(1,1-Dimethyl-prop-2-ynyl)-2-(3-ethyl-8-methyl-quinolin-6-yloxy)-2-methylsulfanyl-acetamide | 92 - 93 |
| 19 | 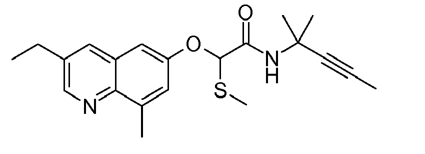 | N-(1,1-Dimethyl-but-2-ynyl)-2-(3-et hyl-8-methyl-quinolin-6-yloxy)-2-methylsulfanyl-acetamide | 166 - 167 |
| 20 | 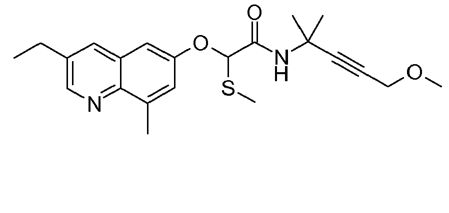 | 2-(3-Ethyl-8-methyl-quinolin-6-yloxy)-N-(4-methoxy-1,1-dimethyl-but-2-ynyl)-2-methylsulfanyl-acetamide | 137 - 138 |
| 21 | 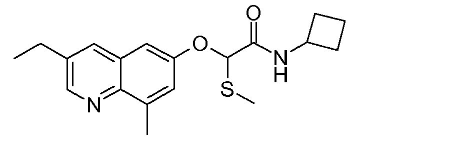 | N-Cyclobutyl-2-(3-ethyl-8-methyl-quinolin-6-yloxy)-2-methylsulfanyl-acetamide | 157 - 158 |
| 22 | 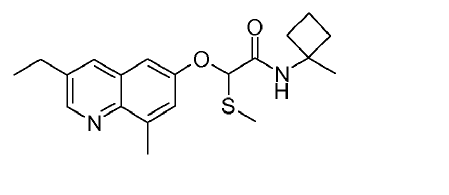 | 2-(3-Ethyl-8-methyl-quinolin-6-yloxy)-N-(1-methyl-cyclobutyl)-2-methylsulfanyl-acetamide | 150-151 |
| 23 | 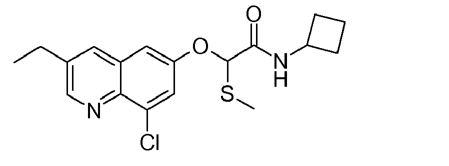 | 2-(8-Chloro-3-ethyl-quinolin-6-yloxy)-N-cyclobutyl-2-methylsulfanyl-ac etamide | 154 - 156 |
| 24 | 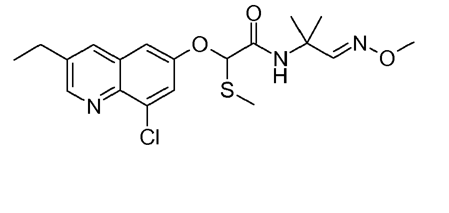 | 2-(8-Chloro-3-ethyl-quinolin-6-yloxy)-N-(2-methoxyimino-1,1-dimethyl-e thyl)-2-methylsulfanyl-acetamide | 142 - 144 |
| 25 | 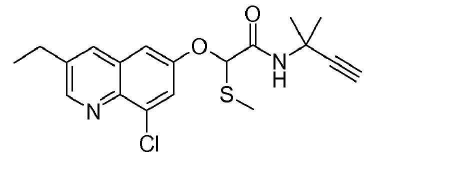 | 2-(8-Chloro-3-ethyl-quinolin-6-yloxy)-N-(1,1-dimethyl-prop-2-ynyl)-2-m ethylsulfanyl-acetamide | 149 - 151 |
| 26 | 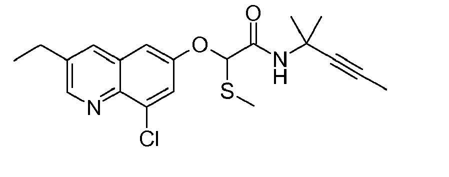 | 2-(8-Chloro-3-ethyl-quinolin-6-yloxy)-N-(1,1-dimethyl-but-2-ynyl)-2-me thylsulfanyl-acetamide | 186 - 188 |
| 27 | 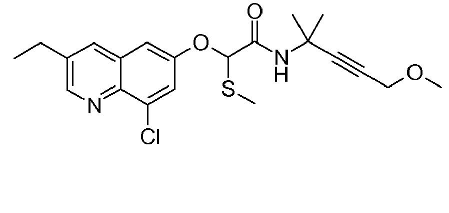 | 2-(8-Chloro-3-ethyl-quinolin-6-yloxy)-N-(4-methoxy-1,1-dimethyl-but-2-ynyl)-2-methylsulfanyl-acetamide | 139 - 142 |
| 28 | 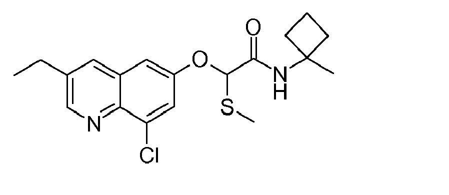 | 2-(8-Chloro-3-ethyl-quinolin-6-yloxy)-N-(1-methyl-cyclobutyl)-2-methylsulfanyl-acetamide | 148 - 150 |
| 29 | 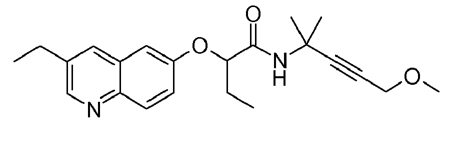 | 2-(3-Ethyl-quinolin-6-yloxy)-N-(4-methoxy-1,1-dimethyl-but-2-ynyl)-but yramide | 99-101 |
| 30 | 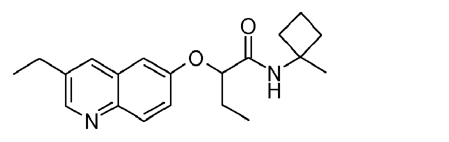 | 2-(3-Ethyl-quinolin-6-yloxy)-N-(1-methyl-cyclobutyl)-butyramide | 110-112 |
| 31 | 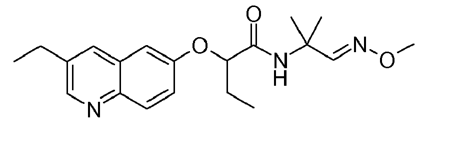 | 2-(3-Ethyl-quinolin-6-yloxy)-N-(2-methoxy-imino-1,1-dimethyl-ethyl)-butyramide | 125 - 127 |
| 32 | 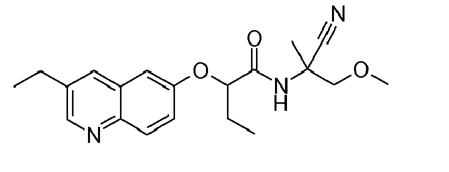 | N-(1-Cyano-2-methoxy-1-methyl-ethyl )-2-(3-ethyl-quinolin-6-yloxy)-butyramide | 114 - 117 |
| 33 | 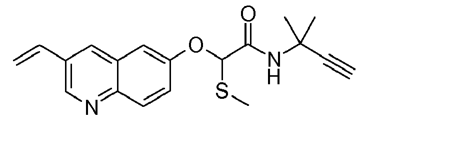 | N-(1,1-Dimethyl-prop-2-ynyl)-2-methylsulfanyl-2-(3-vinyl-quinolin-6-yloxy)-acetamide | 142 - 144 |
| 34 | 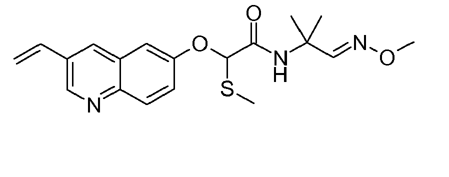 | N-(2-Methoxyimino-1,1-dimethyl-ethy I)-2-methylsulfanyl-2-(3-vinyl-quin olin-6-yloxy)-acetamide | 114 - 116 |
| 35 | 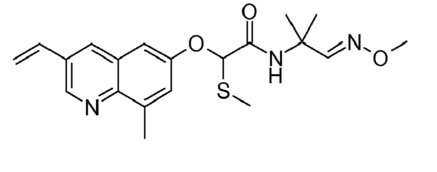 | N-(2-Methoxyimino-1,1-dimethyl-ethy I)-2-methylsulfanyl-2-(8-methyl-3-v inyl-quinolin-6-yloxy)-acetamide | 87 - 89 |

### Example 3

This Example illustrates the fungicidal properties of compounds of formula (I).

Compounds were tested in DMSO solutions against a set of standard screening pathosystems as exemplified below.

Leaf disc tests:
Leaf disks of various plant species (diameter 14 mm) are cut from plants grown in the greenhouse. The cut leaf disks are placed in multiwell plates (24-well format) onto water agar. Immediately after cutting the leaf disks are sprayed with a test solution.
Compounds to be tested are prepared as DMSO solutions (max. 10 mg/ml). Just before spraying the solutions are diluted to the appropriate concentrations with 0.025% Tween20. After drying, the leaf disks are inoculated with a spore suspension of the appropriate pathogenic fungus.

After an incubation time of 3-7 days after inoculation at defined conditions (temp, rH, light, etc.) according to the respective test system, the activity of the test compound is assessed as antifungal activity.

Liquid culture tests:
Mycelia fragments or conidia suspensions of a fungus, prepared either freshly from liquid cultures of the fungus or from cryogenic storage, are directly mixed into nutrient broth. DMSO solutions of the test compound (max. 10 mg/ml) is diluted with 0.025% Tween20 by factor 50 and 10 pl of this solution is pipetted into a microtiter plate (96-well format) and the nutrient broth containing the fungal spores/mycelia fragments is then added to give an end concentration of the tested compound. The test plates are incubated at 24 °C and 96% rH in the dark. The inhibition of fungal growth is determined photometrically after 2 - 6 days and antifungal activity is calculated.

### Phytophthora infestans / tomato / leaf disc preventative (late blight)

Tomato leaf disks are placed on water agar in multiwell plates (24-well format) and sprayed with the formulated test compound diluted in water. The leaf disks are inoculated with a spore suspension of the fungus 1 day after application. The inoculated leaf disks are incubated at 16°C and 75% rh under a light regime of 24 h darkness followed by 12 h light / 12 h darkness in a climate cabinet and the activity of a compound is assessed as percent disease control compared to untreated when an appropriate level of disease damage appears in untreated check leaf disks (5 - 7 days after application).

Compounds 1, 2, 3, 4, 5, 6, 7, 8, 9, 10, 12, 13, 14, 15, 16, 17, 18, 19, 20, 21, 22, 24, 25, 27, 29, 30, 31, 33, 34 and 35 from Table 37 according to the invention at 200 ppm inhibit fungal infestation in this test to at least 80 %, while under the same conditions untreated control plants are infected by the phytopathogenic fungi to over 80 %.

### Plasmopara viticola / grape / leaf disc preventative (late blight)

Grape vine leaf disks are placed on water agar in multiwell plates (24-well format) and sprayed with the formulated test compound diluted in water. The leaf disks are inoculated with a spore suspension of the fungus 1 day after application. The inoculated leaf disks are incubated at 19°C and 80% rh under a light regime of 12 h light / 12 h darkness in a climate cabinet and the activity of a compound is assessed as percent disease control compared to untreated when an appropriate level of disease damage appears in untreated check leaf disks (6 - 8 days after application).

Compounds 2, 3, 6, 7, 8, 9, 12, 13, 14, 15, 16, 17, 18, 19, 20, 21, 22, 24, 25, 27, 28, 33, 34 and 35 from Table 37 according to the invention at 200 ppm inhibit fungal infestation in this test to at least 80 %, while under the same conditions untreated control plants are infected by the phytopathogenic fungi to over 80 %.

### Blumeria qraminis f. sp. tritici (Erysiphe graminis f. sp. tritici) / wheat / leaf disc preventative (Powdery mildew on wheat)

Wheat leaf segments cv. Kanzler are placed on agar in a multiwell plate (24-well format) and sprayed with the formulated test compound diluted in water. The leaf disks are inoculated by shaking powdery mildew infected plants above the test plates 1 day after application. The inoculated leaf disks are incubated at 20°C and 60% rh under a light regime of 24 h darkness followed by 12 h light / 12 h darkness in a climate chamber and the activity of a compound is assessed as percent disease control compared to untreated when an appropriate level of disease damage appears on untreated check leaf segments (6 - 8 days after application).

Compounds 1, 2, 3, 4, 6, 7, 8, 9, 10, 11, 12, 13, 14, 15, 16, 18, 24, 27, 28, 29, 30, 31, 32, 33, 34 and 35 from Table 37 according to the invention at 200 ppm inhibit fungal infestation in this test to at least 80 %, while under the same conditions untreated control plants are infected by the phytopathogenic fungi to over 80 %.

### Phaeosphaeria nodorum (Septoria nodorum) / wheat / leaf disc preventative (Glume blotch)

Wheat leaf segments cv. Kanzler are placed on agar in a multiwell plate (24-well format) and sprayed with the formulated test compound diluted in water. The leaf disks are inoculated with a spore suspension of the fungus 2 days after application. The inoculated test leaf disks are incubated at 20°C and 75% rh under a light regime of 12 h light / 12 h darkness in a climate cabinet and the activity of a compound is assessed as percent disease control compared to untreated when an appropriate level of disease damage appears in untreated check leaf disks (5 - 7 days after application).

Compounds 8, 10, 14, 16, 18, 25, 33, 34 and 35 from Table 37 according to the invention at 200 ppm inhibit fungal infestation in this test to at least 80 %, while under the same conditions untreated control plants are infected by the phytopathogenic fungi to over 80 %.

### Pythium ultimum / liquid culture (seedling damping off)

Mycelia fragments and oospores of a newly grown liquid culture of the fungus are directly mixed into nutrient broth (PDB potato dextrose broth). After placing a (DMSO) solution of test compound into a microtiter plate (96-well format), the nutrient broth containing the fungal mycelia/spore mixture is added. The test plates are incubated at 24°C and the inhibition of growth is determined photometrically 2-3 days after application.

Compounds 1, 2, 3, 4, 5, 6, 7, 8, 9, 10, 11, 12, 13, 14, 15, 16, 17, 18, 19, 20, 21, 22, 24, 28, 29, 30, 31, 33, 34 and 35 from Table 37 according to the invention at 200 ppm inhibit fungal infestation in this test to at least 80 %, while under the same conditions untreated control plants are infected by the phytopathogenic fungi to over 80 %.

## Claims

1. A compound of the general formula (1) 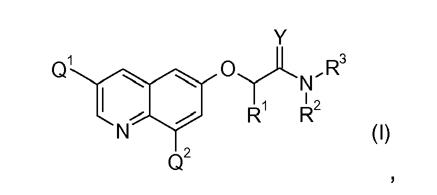 wherein
Q¹ is methyl, ethyl, n-propyl, isopropyl, vinyl or propenyl;
Q² is hydrogen, fluoro, chloro or methyl;
R¹ is ethyl, methoxy or methylthio;
R² is hydrogen or methyl;
R³ is -CR⁴R⁵R⁶;
R⁴ and R⁵, independently of each other, are hydrogen, methyl, ethyl, isopropyl, t-butyl, vinyl, ethynyl, cyano or methoxymethyl, or
R⁴ and R⁵ together with the carbon atom to which they are attached form a 3- to 5-membered carbocyclic ring, which is optionally substituted by methyl;
R⁶ is hydrogen, formyl, methyl, ethyl, 2-fluoroethyl, vinyl, ethynyl, prop-1-ynyl, but-1-ynyl, cyano, cyclopropyl, methoxymethyl, ethoxymethyl, -CH=NOMe, -CH=NOEt or - C=CCH₂OMe; and
Y is oxygen or sulfur;
or a salt or a N-oxide thereof.

2. A compound according to claim 1, wherein Q¹ is methyl, ethyl, vinyl or propenyl.

3. A compound according to claim 2, wherein Q¹ is methyl or vinyl.

4. A compound according to claim 1, wherein Q² is hydrogen or methyl.

5. A compound according to claim 1, wherein R¹ is methylthio.

6. A compound according to claim 1, wherein R² is hydrogen.

7. A compound according to claim 1, wherein R⁴ and R⁵, independently of each other, are methyl, ethyl, vinyl, ethynyl or cyano.

8. A compound according to claim 1, wherein one of R⁴ and R⁵ is methyl and the other of R⁴ and R⁵ is ethyl, vinyl, ethynyl or cyano.

9. A compound according to claim 1, wherein R⁴ and R⁵ together with the carbon atom to which they are attached form a cyclobutyl.

10. A compound according to claim 1, wherein R⁶ is methyl, ethynyl, -CH=NOMe or -C=CCH₂OMe.

11. A compound according to claim 1, wherein Y is oxygen.

12. A compound according to claim 1, wherein Q¹ and Q² are methyl and R¹ is methylthio.

13. A compound according to claim 1, wherein Q¹ is methyl or vinyl; Q² is hydrogen, chloro or methyl; R¹ is ethyl, methoxy or methylthio; R² is hydrogen; R³ is ―CR⁴R⁵R⁶; R⁴ and R⁵, independently of each other, are hydrogen, methyl, ethyl, isopropyl, t-butyl, vinyl, ethynyl, cyano or methoxymethyl, or R⁴ and R⁵ together with the carbon atom to which they are attached form a cyclobutyl ring, which is optionally substituted by methyl, R⁶ is hydrogen, methyl, ethyl, vinyl, ethynyl, prop-1-ynyl, cyano, methoxymethyl, -CH=NOMe or -C=CCH₂OMe; Y is oxygen.

14. A fungicidal composition comprising a fungicidally effective amount of a compound of formula (1) according to claim 1, a suitable carrier or diluent therefore, and optionally a further fungicidal compound.

15. A method of combating or controlling phytopathogenic fungi which comprises applying a fungicidally effective amount of a compound of formula (I) according to claim 1 or a composition according to claim 14 to a plant, to a seed of a plant, to the locus of the plant or seed or to soil or any other plant growth medium.
